# EUROPEAN PATENT SPECIFICATION

(11) **EP 3 639 892 B1**
(45) Date of publication and mention of the grant of the patent: **04.08.2021**
(21) Application number: 18200423.4
(22) Date of filing: 15.10.2018
(51) Int. Cl.: A61N 5/10

(54) **RADIOTHERAPY SYSTEM AND OPERATING METHOD**
STRAHLENTHERAPIESYSTEM UND BETRIEBSVERFAHREN
SYSTÈME DE RADIOTHÉRAPIE ET PROCÉDÉ DE FONCTIONNEMENT

(43) Date of publication of application: 22.04.2020
(73) Proprietor: Elekta Limited, West Sussex RH10 9RR (GB)
(72) Inventor: BERGFJORD, Per, East Grinstead West Sussex RH19 3EA (GB); LIU, Dr. Rui, 86153 Augsburg (DE); LUTZ, Ulrike, 86899 Landsberg (DE); WINFIELD, Colin, Crawley West Sussex RH11 8PU (GB); WEBER, Florian, 85354 Freising (DE); COSSU, Antonio, 22070 Senna Comasco - COMO (IT); LITTLE, Andrew, Crawley West Sussex RH10 9BL (GB)
(74) Representative: Hamer, Thomas Daniel

(56) References cited:
- EP-A1- 2 926 734
- WO-A1-2006/062872
- WO-A2-2007/098899
- US-A1- 2002 085 668
- US-A1- 2016 023 019

## Description

### Field of the Invention

The present invention relates generally to real-time image guided radiation therapy. In particular, the present invention relates to a radiotherapy system and a method of operating a radiotherapy system in order to verify the isocenter.

### Background of the Invention

Medical imaging is commonly used to assist in the diagnosis and/or treatment of patients. X-Ray imaging is an example of a medical imaging technology that is often performed during the diagnosis and/or treatment of tumors. The treatment of tumors may be performed by using ionizing radiation provided by a linear accelerator (LINAC) generating a radiation beam of electrons and/or protons having particular energies. In such a radiotherapy system, the radiation beam should be aimed as precisely as possible at the target volume or target tissue, namely the tumor, while the adjacent healthy tissue should as far as possible not be irradiated.

Commonly, the radiation beam may be delivered by a radiation source configured to be moved in a circular orbit about the target volume to be irradiated. The radiation source may be moved to different positions in the circular orbit to deliver the radiation beam from these positions to the target volume. In this regard it is preferred that the radiation beam delivered from the different positions intersect in one single point, which is a reference point and which is commonly referred to as isocenter. In other words, the isocenter may be regarded as the AO:TE point in space where radiation beams intersect when the radiation source is rotated during beam generation. In a radiotherapy system, it may therefore be desirable to verify the isocenter as accurately as possible before starting the treatment.

However, even with a previous verification of the isocenter, during operation of the radiotherapy system, the accuracy of verifying the isocenter may be influenced by several factors. For example, the mass of the radiation source to be accelerated, the gravity acting on mechanical parts of the radiotherapy system etc. may be such factors. Accordingly, when moving the radiation source as explained above, deformation of at least the radiation source may occur. Although this deformation should be minor, it may be displace the isocenter and negatively affect the treatment outcome. However, even without such deformation, the isocenter may differ for different photon energies. With a displacing isocenter, a real path of the radiation beam delivered from the radiation source may deviate from its ideal path so that the target volume may not be hit as precisely as it is intended. As a result, radiation may also be delivered to healthy tissue.

Besides of a commonly known, so-called Winston-Lutz test, several approaches for verifying the isocenter are known in the art. For example, US 8 488 862 B2 suggests means to obtain a projection image of a phantom having a plurality of fiducials at known reference positions in a coordinate system associated with the phantom irradiated by a radiotherapy radiation source at a plurality of discrete locations of a trajectory path model. Then, a projection matrix from the projection image corresponding to each discrete location of the trajectory and the actual coordinate of the radiotherapy radiation source in the coordinate system associated with the phantom at each of the discrete locations based on the determined projection matrices are determined. Then, the trajectory path model of the radiotherapy radiation source to the determined actual position of the radiotherapy radiation source at the plurality of discrete locations is correlated. Other approach is described in publication US 2016/0023019.

However, there may be a need to provide a radiotherapy system and/or an operation method for a radiotherapy system that provides an improved verification of the isocenter.

### Summary of the invention

One aspect of the present invention provides a radiotherapy system configured for diagnosing and/or treating a patient. The radiotherapy system may comprise e.g. a linear accelerator (LINAC) system together with an X-Ray imaging system and / or a magnetic resonance (MR) system and / or computed tomography (CT) system.

The radiotherapy system comprises a couch for supporting the patient to be diagnosed and/or treated. The system further comprises a gantry which is configured to be rotatable around a gantry axis and having and/or carrying a radiation source. In particular, the gantry may be moved to different positions in the circular orbit to deliver a radiation beam from these positions to a target volume of the patient. The radiation source may comprise and/or may be referred to as a radiation head, a collimator, such as a multi-leaf-collimator (MLC), etc. In this context, the gantry itself may also be considered as the radiation source. The system further comprises at least one radiation imaging device. The radiation imaging device may be provided as a kV and/or MV imaging device configured to provide 2D, 3D and/or 4D imaging.

The system further comprises a determining or calibration system comprising at least one first optical detection means mounted at the gantry. For example, the first optical detection means may be a camera or the like. Further, the calibration system comprises at least one second optical detection means fixed in a surrounding area of the couch and/or the gantry. For example, the second may be arranged on a wall or a ceiling of treatment room accommodating the radiotherapy system. The calibration system further comprises a set of first fiducial markers which are selectively attachable at the couch at defined positions and configured to be detectable by the first optical detection means. Further, the calibration system comprises a phantom which is selectively attachable at the couch at a defined position, wherein the phantom comprises a set of second fiducial markers configured to be detectable by the second optical detection means and a set of third fiducial markers configured to be detectable by the radiation imaging device. The third fiducials markers may be arranged on defined positions at the phantom and may be visible for the radiation imaging device, such as an MV and/or a kV imager receiver or the like.

The radiotherapy system further comprises a controlling means. The controlling means is at least configured to at least selectively activate the radiation source and rotate the gantry, and, for one or more rotational positions of the gantry, to determine a point of intersection of a beam axis of the radiation source and the gantry axis by linking detection data of at least the first optical detection means, the second optical detection means and/or the radiation imaging device. The point of intersection may be referred to as a (floating) isocenter. The controlling means may comprise a processor, a memory etc. for processing and/or storing e.g. imaging data, calibration data, such as isocenter determination data, or the like. It may be connected to other parts of the systems by suitable data communication lines.

Several advantages may be achieved with this configuration of the radiotherapy system. For example, the (floating) isocenter may be precisely and accurately determined for each single rotational angle, i.e. the current rotational position relative to the target, although the isocenter may be different for different rotational angles due to mechanical deformation caused by the weight of the gantry or the gravitational force. Further, the system may improve precise verification of the (floating) isocenter, while reducing exposure of the patient to X-rays. In particular, a dose calibration, X-Ray imaging calibration and verifying the floating isocenter with gantry rotation calibration may be done by use of the phantom and a one-time gantry rotation, e.g. by rotating the gantry 360° about the gantry axis. In a common system, however, if the target is outside of the tube, patient re-positioning with a movement of the couch would be needed, and afterwards an imaging by use of a radiation imaging device and re-mapping with CT images would start again. Consequently, in a common system, the patient would be exposed to a high level of radiation exposure just in order to be moved into a proper position. In contrast thereto, in the system according to this invention, the optical detection means may allow a table movement without using e.g. X-Rays by using e.g. IR-markers to guide the couch movement, and an extended field of view. Therefore, this invention may provide a non-invasive position and/or isocenter verification, and reduces set-up time of the patient.

In an embodiment of the invention, wherein the controlling means is further configured to control a position and/or orientation of the couch to align it at the determined point of intersection, namely the currently determined (floating) isocenter. The couch may have six degrees of freedom for its own movement and may further be driven by a controllable drive.

Thus, if it is determined that the radiation beam is not (or no more) aligned with the target, this undesirable deviation can be compensated by controllable moving the anyway movable couch. In other words, the system may be configured to follow the current (floating) isocenter.

According to a further embodiment of the invention, the controlling means may further be configured to, based on an image of the phantom captured by the at least one radiation imaging device, calibrate a beam shaper of the radiation source at each rotational position of the gantry. The beam shaper may be a multi-leaf-collimator (MLC) or the like.

Thus, the beam shaper may be adjusted to the respective currently determined (floating) isocenter, especially in real-time, so that even slight deformations of the gantry or other mechanical parts of the radiotherapy system and a resulting floating isocenter can be taken into account.

In another embodiment of the invention, the controlling means may be further configured to, based on an image of the phantom captured by the at least one radiation imaging device, determine in real-time a radiation dose to be delivered by the radiation source at each rotational position of the gantry.

Thus, an on-line dose adaption dependent on the current isocenter may be performed.

In an embodiment of the invention, the controlling means may be further configured to, based on an image of the phantom captured by the at least one radiation imaging device, determine an offset of the gantry at each rotational position of the gantry.

Thus, the (floating) isocenter may be determined even more precisely at each rotational angle of the gantry.

In another embodiment of the invention, the third fiducial markers may be embedded in a material of the phantom to be visible by use of the at least one radiation imaging device.

Thus, since fiducial markers are embedded in the phantom and be visible for MV and/or kV imager receivers, projected dots at 2D plane can be used for calculating the gantry offset at each rotation angle.

According to a further embodiment of the invention, wherein the phantom may be linked to the at least one first optical detection means. It may be linked via suitable data communication lines, the controlling means and/or a software-based control.

Thus, determining the current (floating) isocenter may be performed even more precisely.

In another embodiment of the invention, the phantom, via the second optical detection means, may be linked to at last one absolute position in a free three-dimensional space.

Thus, determining the current (floating) isocenter may be performed even more precisely. The phantom is not only used for geometry calibration but may be also used for beam and imaging calibration.

According to a further embodiment of the invention, the phantom may further comprise a further set of first fiducial markers configured to be detectable by the at least one first optical detection means.

Thus, the phantom may be linked, via the first fiducial markers, to the first optical detection means.

In another embodiment of the invention, a further first optical detection means, or a similar means, may be mounted at the at least one radiation imaging device to be arranged opposite to the first optical detection means mounted at the gantry.

Thus, corresponding first fiducial markers may be detectable even if the gantry is currently located on a side facing away from the couch top.

In an embodiment of the invention, further first fiducial markers may be selectively attachable at a bottom side of the couch.

Thus, the first optical detection means may detect one of the first fiducial markers even if the gantry is currently located on a side facing away from the couch top or, respectively, facing the bottom side of the couch.

According to a further embodiment of the invention, wherein first fiducial markers are carried by at least one frame structure which can be mounted along a top side of the couch at different attachment positions.

Thus, even if the phantom has already been removed before loading the patient on the couch top, the target tumor position can be well determined based on the kV imager and previous imaging calibration, since the reference frame structure is still detectable and/or trackable by the first optical detection means mounted at least at the gantry.

In another embodiment of the invention, the first optical detection means may be an IR-camera.

Thus, a cost-effective and small-sized possibility for the marker detection is possible.

According to a further embodiment of the invention, the second optical detection means may be a laser device. For example, the second optical detection means may be a laser tracker or the like.

Thus, the phantom is linkable with pre-defined absolute position(s) at 3D free space.

In another embodiment of the invention, a further set of the first fiducial markers may be provided via a support structure attachable to a patient and wherein these first fiducial markers are linked to the first fiducial markers attached to the couch.

Thus, co-registering with the other first fiducial markers, e.g. mounted at the reference frame structure, is possible which may allow real-time motion detection of the patient. Ideally, this information may be provided to the controlling means to control calibration and/or treatment accordingly.

According to an embodiment of the invention, the controlling means may further be configured, for the one or more rotational positions of the gantry, to determine a current or predicted couch position and, via the first optical detection means and the first fiducial markers, to determine a current or predicted position of the gantry relative to the determined current or predicted couch position.

Thus, a collision between the gantry and the couch can be avoided. In particular, since the first optical detection means is mounted at the gantry, a distance between e.g. the couch top and the gantry may be determined, at each gantry angle during calibration. At each gantry angle, even all possible locations and/or positions of the couch may be known, e.g., from a simulation from a 3D CAD model or the like. Since the first fiducial markers are arranged at a pre-defined position on the couch top, a minimum safety distance between the first optical detection means and the first fiducial markers may be calculated and/or simulated. After calibration, a table-look-up approach can be applied for each optical detection means.

In another embodiment of the invention, the controlling means may be further configured to, based on determining a mechanical center of the gantry via the first optical detection means, determine a current or predicted angular velocity and/or acceleration.

Thus, the first optical detection means mounted at the gantry may be used to determine the angular velocity and/or acceleration of the rotating gantry. In other words, after calibration, the gantry mechanical center is known by use of the first optical detection means, for each gantry and/or couch position. Since the weight or mass of the gantry, e.g. its head and arm, is known, e.g. from a 3D CAD model or the like, it may be determined what level of energy is needed to drive or stop the gantry to a target angle with a desired speed and acceleration. For example, the gantry is currently at a 6 o'clock position and it desired to stop it at a 12:00 o'clock position, gravity, inertia and the like may be used to set optimized settings for acceleration, drive and/or braking.

An aspect of the invention provides a method of operating a radiotherapy system, wherein the method comprises the following steps:
- providing a set of first fiducial markers at the couch of the radiotherapy system at defined positions,
- providing the phantom at the couch at a defined position, wherein the phantom comprises the set of second fiducial markers and the set of third fiducial markers,
- detecting the first fiducial markers by the first optical detection means mounted on the gantry which is configured to be rotatable around a gantry axis and having a radiation source,
- detecting the second fiducial markers by the second optical detection means fixed in the surrounding area of the couch and/or the gantry,
- detecting the third fiducial markers by the radiation imaging device of the radiotherapy system,
- controlling the radiation source to be at least selectively activated and controlling the gantry to be rotated, and
- for one or more rotational positions of the gantry, determining a point of intersection of a beam axis of the radiation source and the gantry axis by linking detection data of at least the first optical detection means, the second optical detection means and/or the radiation imaging device.

In other words, the first fiducial markers and the phantom may be provided at e.g. the couch top to have a reproducible position. The second optical detection means may be arranged on e.g. a wall or ceiling of a room accommodating the radiotherapy system, so as to link with pre-defined absolute positions in 3D free space. Then, the radiation source is activated to generate a radiation beam while the gantry is controlled to be rotated, e.g. about 360° about the gantry axis, so as to link the phantom to the first optical detection means and/or the radiation imaging device, such as a MV image receiver panel, at each gantry angle position. It is noted that, since the third fiducial markers are arranged precisely at the phantom and visible for e.g. MV and/or kV imager receivers, projected dots at 2D plane may be used for calculating a gantry offset at each rotation angle. Therefore, the (floating) isocenter may be determined at each gantry angle. Similarly, such information can also be linked to the first optical detection means during calibration, because the introduced first fiducial markers are adapted to provide a large field of view (FoV) as compared to e.g. a common Winston-Lutz ball approach.

In an embodiment of the invention, the phantom image may be used to calibrate a beam shaper of the radiation source, such as a MLC, and to determine a proper X-Ray imaging at each angle.

In an embodiment of the invention, the phantom is removed from the couch and the patient is loaded.

Thus, since the first fiducial markers may still be tracked with the one or more first optical detection means mounted on the gantry, the target tumor position may be precisely determined based on the kV imager and previous imaging calibration. By using more than one first fiducial markers a single-fault problem may be avoided, so that the above system may also be suitable for e.g. multi-fraction non-coplanar LINAC treatment. In addition, further first fiducial markers may be arranged at the patient body to co-register with the first fiducial markers provided at the couch such that real-time motion of the patient may be monitored.

According to an embodiment, the couch may be driven to follow the determined (floating) isocenter at each gantry angle.

In another embodiment, the radiation source may be controlled in real-time to adjust the beam shaping on basis of the determined (floating) isocenter. In addition or alternatively, an on-line dose adaption of the radiation dose may be performed.

It should be noted that embodiments as described above may be combined with respect to each other so as to gain a synergetic effect, which may extend over the separate technical effects of the single features. Likewise, the above method can be modified by the embodiments of the above radiotherapy system and vice versa.

### Brief description of the figures

Exemplary embodiments of the invention will be described in the following with reference to the following figures.
- Figure 1: shows a perspective view of a radiotherapy system,
- Figure 2: shows a side view of a phantom,
- Figure 3: shows a perspective view of the radiotherapy system of Figure 1, wherein a phantom has been removed and replaced by a patient to be diagnosed and/or treated,
- Figure 4: shows a schematic front view of the radiotherapy system
- Figure 5: shows a flow chart of a method of operating a radiotherapy system.

The figures are merely schematic representations and serve only to illustrate the invention. Identical or equivalent elements are consistently provided with the same reference signs.

### Detailed description of exemplary embodiments

In the following, a detailed description of exemplary embodiments will be given to explain the invention in more detail.

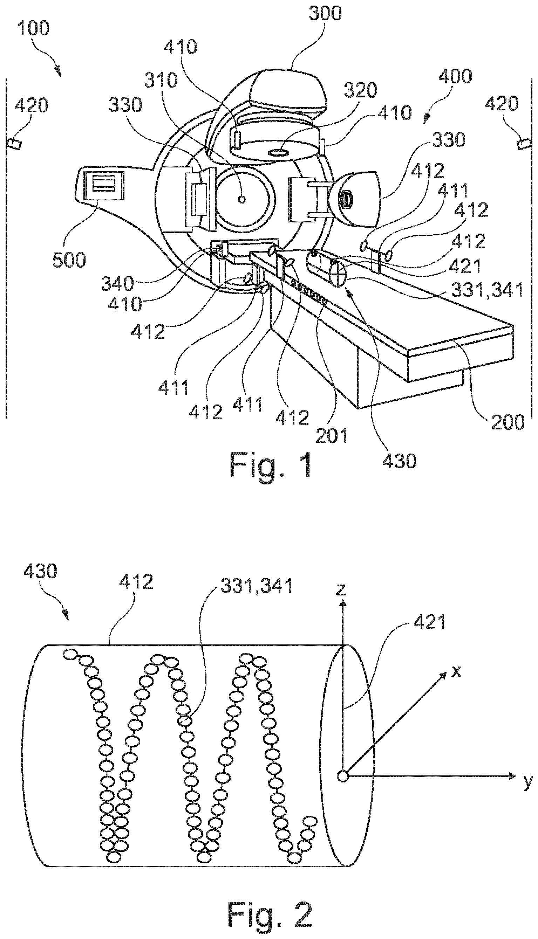

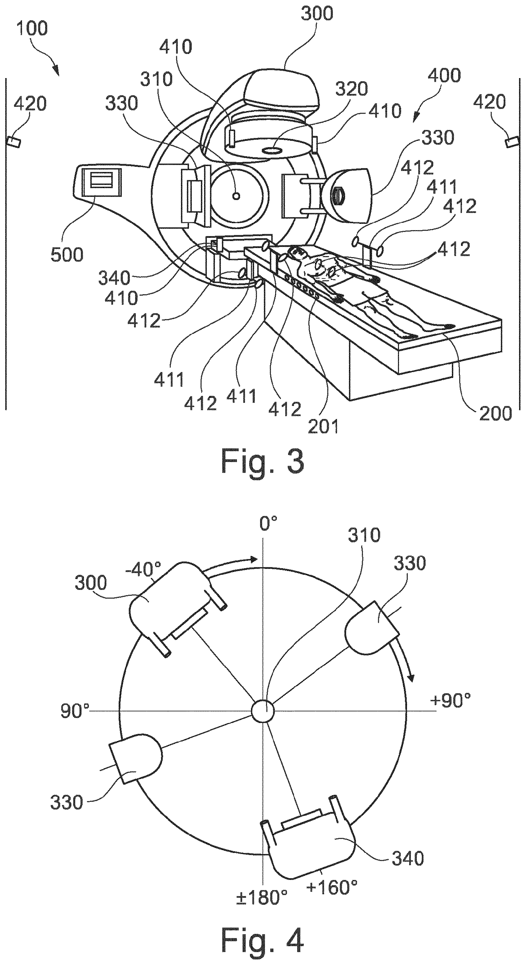

Figure 1 illustrates schematically a radiotherapy system 100, which, in some embodiments, may be configured as a linear accelerator (LINAC) radiotherapy system. The system 100 comprises a couch 200 configured to support a recumbent patient on a couch top (see Figure 3). The couch is electronically controllable and movable with six degrees of freedom. The system 100 further comprises a gantry 300 having a gantry arm 311 and a gantry head 312. The gantry 300 is rotatable around a gantry axis 310 and has a radiation source 320. The radiation source 320 may comprises at least one beam shaper, such as a multi leaf collimator (MLC), and may provide or produce X-rays and/or high energy electrons or photons in form of a radiation beam for medicinal purposes in radiation therapy. The system 100 further comprises at least one radiation imaging device 330, 340, wherein, in some embodiments, a first radiation imaging device 330 may be configured as a kV imager and a second radiation imaging device 340 may be configured as a MV imager. In the embodiment shown in Figure 1, the first radiation imaging device 330 comprises an imager radiation source and an imager receiver panel. Likewise, but offset from the first radiation imaging device 330, the second radiation imaging device 340 comprises an imager radiation source and an imager receiver panel.

As illustrated in Figure 1, the system 100 further comprises a determining or calibration system 400 configured to at least determine an isocenter, which may be regarded as a point of intersection of a radiation beam axis of the radiation source 320 and the gantry axis 310. The determined (floating) isocenter may be the location in which the target volume to be irradiated is to be arranged. At the gantry 300, at least one first optical detection means 410 is mounted, wherein, in this embodiment, two exemplary first optical detection means 410 in form of infrared / IR cameras are provided. The first optical detection means 410 are fixedly mounted near the radiation beam outlet of the radiation source 320 so that they may participate any movement, e.g. the rotation of the gantry 300, but also any deformation of the gantry 300, e.g. of the gantry arm 311 and/or gantry head 312, due to its weight, gravity, centrifugal force, etc. In some embodiments, at least one further first optical detection means 410 may be arranged at a side of the system 100 opposing the first optical detection means 410 mounted at the gantry 300. As illustrated in Figure 1, the further first optical detection means 410 are mounted at the receiver panel of the second radiation imaging device 340.

In a surrounding area of the couch 200 and/or the gantry 300, at least one second optical detection means 420 of the system 100, in particular the system 400 is fixed in place. The surrounding area may be a wall, like in Figure 1, a ceiling etc. of a room accommodating the system 100. By way of example, only two second optical detection means 420 are shown, however, one, three, four, five, six or more can be provided and aligned to the system 100, in particular the couch 200 or the couch top. The second optical detection means may be a laser tracker.

Further, the system 100, in particular the system 400 comprises a set of first fiducial markers 412 which are selectively attachable and detachable at the couch 200 at defined positions and configured to be detectable by the first optical detection means 410. In some embodiments, the couch 200 comprises several attachment positions 201, e.g. engaging holes, so that the first fiducial markers 412 may be mounted along a top side of the couch 200 at different positions. In some embodiments, the first fiducial markers 412 are attached to e.g. a reference frame structure 411 which is mountable at an edge region of the couch 200 and extends away from the couch top. In some embodiments, on a bottom side of the couch 200, a further set of first fiducial markers 412 may be attached to a further reference frame structure 411.

The system 100, in particular the system 400 further comprises a phantom 430 which is selectively attachable to and detachable from the couch 200 at a defined position. The couch top may comprise reference points, lines etc. to facilitate alignment of the phantom, as indicated in Figure 1 by a dashed line. In Figure 1, the phantom 430 is a tube and comprises a set of second fiducial markers 421 configured to be detectable by the second optical detection means 420, e.g. the laser tracker. In some embodiments, the phantom 430 may be linked to at last one absolute position in free three-dimensional space via the second optical detection means 420 is linked. The phantom 430 further comprises a set of third fiducial markers 331, 341 configured to be detectable by the radiation imaging device 330 and/or 340. In some embodiments, the third fiducial markers 331, 341 may be embedded in a material of the phantom 430 to be visible by use of the at least one of the radiation imaging devices 330, 340. In some embodiments, the phantom 430 may further comprise a further set of the first fiducial markers 412 so that the phantom 430 may be linked to the at least one first optical detection means 410.

The system 100 further comprises a controlling means 500 which may be an electronic device comprising a processor, a physical memory etc. In particular for calibrating the system 100 and/or determining a (floating) isocenter of the system 100, the controlling means 500 is configured to selectively activate the radiation source 320 and rotate the gantry 300 about the gantry axis 310, around any angle section or e.g. 360°. This rotation may be also be referred to as a movement in a circular orbit about a target volume to be irradiated. Further, the controlling means 500 is configured to, for one or more rotational positions of the gantry 300 relative to the starting point of rotation, determine a point of intersection, e.g. the (floating) isocenter, of the beam axis of the radiation source 320 and the gantry axis 310 by linking detection data of at least the first optical detection means 410, the second optical detection means 420 and/or the radiation imaging device 330, 340.

In some embodiments, the controlling means 500 may be further configured to control a position and/or orientation of the couch 200 to align it at the determined point of intersection, e.g. the (floating) isocenter. The controlling means 500 may further configured to, based on an image of the phantom 430 captured by the at least one radiation imaging device 330, 340, calibrate the beam shaper of the radiation source 320 at each rotational position of the gantry 300. In addition or alternatively, the controlling means 500 may be further configured to, based on an image of the phantom 430 captured by the at least one radiation imaging device 330, 340, determine a radiation dose to be delivered by the radiation source 320 at each rotational position of the gantry 300. Further, in some embodiments, the controlling means 500 may further be configured to, based on an image of the phantom 430 captured by the at least one radiation imaging device 330, 340, determine an offset of the gantry 300 at each rotational position of the gantry 300. In some embodiments, the controlling means 500 may further be configured, for the one or more rotational positions of the gantry 300, to determine a current or predicted position of the couch 200. If the position of the couch 200 is known, via the first optical detection means 410 and the first fiducial markers 412, the controlling means 500 may determine a current or predicted position of the gantry 300 relative to the determined current or predicted position of the couch 200. Further, in some embodiments, the controlling means 500 may be further configured to, based on determining a mechanical center of the gantry 300 via the first optical detection means 410, determine a current or predicted angular velocity and/or acceleration.

Figure 2 shows the phantom 430 as a single part which is exemplarily formed as the tube and comprises the set of second fiducial markers 421 configured to be detectable by the second optical detection means 420, e.g. the laser tracker, the set of third fiducial markers 331, 341 embedded in a material of the phantom 430 to be visible by use of the at least one of the radiation imaging devices 330, 340 and configured to be detectable by the radiation imaging device 330 and/or 340. In this exemplarily embodiment, the phantom 430 further comprises the further set of the first fiducial markers 412 so that the phantom 430 may be linked to the at least one first optical detection means 410.

In Figure 3, the phantom 430 has been removed from the top side of the couch 200. Instead, as schematically indicated, a patient has been placed on the top side of the couch 200. In some embodiments, the patient may wear a support structure 413, e.g. a textile part or the like, where a further set of the first fiducial markers 412 is attached. This further set of the first fiducial markers 412 is linked to the other first fiducial markers 412 attached to the couch 200. In some embodiments, the controlling means 500 may be configured to detect motion of the patient via the first optical detecting means 410 by detecting e.g. deviations in positional deviations between the patient-sided first fiducial markers 412 and the couch-sided first fiducial markers 412.

Figure 4 shows a schematic front view of the system 100, wherein the couch 200 is hidden for better illustration. As explained above, the controlling means 500 may be configured to control the angular velocity and/or acceleration of the gantry 300. For that, the first optical detection means 410 mounted at the gantry 300 may be used to determine the angular velocity and/or acceleration of the rotating gantry 300. After calibration of the system 100, the gantry 300 mechanical center is already known by use of the first optical detection means 410, for each gantry and/or couch position. Since the weight or mass of the gantry 300, e.g. its gantry head and arm, is known, e.g. from a 3D CAD model, trigonometry, or the like, it may be determined what level of energy is needed to drive or stop the gantry 300 to a target angle with a desired speed and acceleration. In the example according to Figure 4, the gantry 300 is to be moved from its starting point at about -40° to its end point at about +160°.

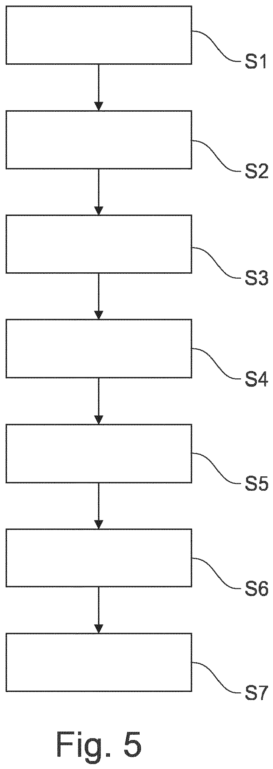

With reference to the flow chart shown in Figure 5, an operation and/or a calibration of the radiotherapy system 100 may be as described below.

In a first step S1, the set of first fiducial markers 412 is provided, e.g. mounted, at the couch 200 at the defined positions. These positions may vary in dependency from the patient, the target volume, the location of the target volume etc. In a further step S2, the phantom 430 is provided, e.g. mounted and/or aligned with a reference mark or the like, at the couch 200 at a defined position, wherein the phantom 430 comprises the set of second fiducial markers 421 and the set of third fiducial markers 331, 341. In a step S3, the first fiducial markers 412 are detected by the first optical detection means 410 mounted on the gantry 300 which is configured to be rotatable around the gantry axis 310 and carrying the radiation source 320. In a step S4, the second fiducial markers 421 are detected by the second optical detection means 420 fixed in a surrounding area of the couch 200 and/or the gantry 300. In a step S5, the third fiducial markers 331, 431 are detected by a radiation imaging device 330, 340 of the radiotherapy system 100. In a step S6, the radiation source 320 is controlled to be selectively activated and the gantry 320 is controlled to be rotated. In a step S7, for one or more rotational positions of the gantry 300, the point of intersection of the beam axis of the radiation source 320 and the gantry axis 310, e.g. the (floating) isocenter, is determined by linking detection data of at least the first optical detection means 410, the second optical detection means 420 and/or the radiation imaging device 330, 340.

## Claims

1. A radiotherapy system (100), comprising
- a couch (200),
- a gantry (300) which is configured to be rotatable around a gantry axis (310) and having a radiation source (320),
- at least one radiation imaging device (330, 340),
- a calibration system (400) comprising
- at least one first optical detection means (410) mounted at the gantry (300),
- at least one second optical detection means (420) fixed in a surrounding area of the couch (200) and/or the gantry (300),
- a set of first fiducial markers (412) which are selectively attachable at the couch (200) at defined positions and configured to be detectable by the first optical detection means (410), and
- a phantom (430) which is selectively attachable at the couch (200) at a defined position, wherein the phantom (430) comprises a set of second fiducial markers (421) configured to be detectable by the second optical detection means (420) and a set of third fiducial markers (331, 341) configured to be detectable by the radiation imaging device (330, 340), and
- a controlling means (500) which is configured to selectively activate the radiation source (320) and rotate the gantry (300), and, for one or more rotational positions of the gantry (300), to determine a point of intersection of a beam axis of the radiation source (320) and the gantry axis (310) by linking detection data of at least the first optical detection means (410), the second optical detection means (420) and/or the radiation imaging device (330, 340).

2. The radiotherapy system (100) of claim 1, wherein the controlling means (500) is further configured to control a position and/or orientation of the couch (200) to align it at the determined point of intersection.

3. The radiotherapy system (100) of claim 1 or 2, wherein the controlling means (500) is further configured to, based on an image of the phantom (430) captured by the at least one radiation imaging device (330, 340), calibrate a beam shaper of the radiation source (320) at each rotational position of the gantry (300).

4. The radiotherapy system (100) of any one of the preceding claims, wherein the controlling means (500) is further configured to, based on an image of the phantom (430) captured by the at least one radiation imaging device (330, 340), calibrate the radiation imaging device (330, 340) at each rotational position of the gantry (300).

5. The radiotherapy system (100) of any one of the preceding claims, wherein the controlling means (500) is further configured to, based on an image of the phantom (430) captured by the at least one radiation imaging device (330, 340), determine an offset of the gantry (300) at each rotational position of the gantry (300).

6. The radiotherapy system (100) of any one of the preceding claims, wherein the third fiducial markers (331, 341) are embedded in a material of the phantom (430) to be visible by use of the at least one radiation imaging device (330, 340).

7. The radiotherapy system (100) of any one of the preceding claims, wherein the phantom (430) is linked to the at least one first optical detection means (410).

8. The radiotherapy system (100) of any one of the preceding claims, wherein the phantom (430), via the second optical detection means (420), is linked to at last one absolute position in a free three-dimensional space.

9. The radiotherapy system (100) of any one of the preceding claims, wherein the phantom (430) further comprises a further set of first fiducial markers (412) configured to be detected by the at least one first optical detection means (410).

10. The radiotherapy system (100) of any one of the preceding claims, wherein a further first optical detection means (410) is mounted at the at least one radiation imaging device (330, 340) to be arranged opposite to the first optical detection means (410) mounted at the gantry (300).

11. The radiotherapy system (100) of any one of the preceding claims, wherein further first fiducial markers (412) are selectively attachable at a bottom side of the couch (200).

12. The radiotherapy system (100) of any one of the preceding claims, wherein first fiducial markers (412) are carried by at least one frame structure (411) which can be mounted along a top side of the couch (200) at different attachment positions (201).

13. The radiotherapy system (100) of any one of the preceding claims, wherein the first optical detection means (410) is an IR-camera.

14. The radiotherapy system (100) of any one of the preceding claims, wherein the second optical detection means (420) is a laser device.

15. The radiotherapy system (100) of any one of the preceding claims, wherein a further set of the first fiducial markers (412) is provided via a support structure (413) attachable to a patient and wherein these first fiducial markers (412) are linked to the first fiducial markers (412) attached to the couch (200).

16. The radiotherapy system (100) of any one of the preceding claims, wherein the controlling means (500) is further configured, for the one or more rotational positions of the gantry (300), to determine a current or predicted couch position and, via the first optical detection means (410) and the first fiducial markers (412), to determine a current or predicted position of the gantry (300) relative to the determined current or predicted couch (200) position.

17. The radiotherapy system (100) of any one of the preceding claims, wherein the controlling means (500) is further configured to, based on determining a mechanical center of the gantry (300) via the first optical detection means (410), determine a current or predicted angular velocity and/or acceleration of the gantry (300).

18. A method of operating a radiotherapy system (100), comprising the steps:
- providing a set of first fiducial markers (412) at a couch (200) of the radiotherapy system (100) at defined positions,
- providing a phantom (430) at the couch (200) at a defined position, wherein the phantom (430) comprises a set of second fiducial markers (421) and a set of third fiducial markers (331, 341),
- detecting the first fiducial markers (412) by a first optical detection means (410) mounted on a gantry (300) which is configured to be rotatable around a gantry axis (310) and having a radiation source (320),
- detecting the second fiducial markers (421) by a second optical detection means (420) fixed in a surrounding area of the couch (200) and/or the gantry (300),
- detecting the third fiducial markers (331, 431) by a radiation imaging device (330, 340) of the radiotherapy system (100),
- controlling the radiation source (320) to be selectively activated and controlling the gantry (320) to be rotated, and
- for one or more rotational positions of the gantry (300), determining a point of intersection of a beam axis of the radiation source (320) and the gantry axis (310) by linking detection data of at least the first optical detection means (410), the second optical detection means (420) and/or the radiation imaging device (330, 340).

## Patentansprüche

1. Strahlentherapiesystem (100), umfassend:
- eine Liege (200),
- eine Gantry (300), die so ausgestaltet ist, dass sie um eine Gantry-Achse (310) rotierbar ist und eine Strahlungsquelle (320) aufweist,
- mindestens eine Strahlungsbildgebungsvorrichtung (330, 340),
- ein Kalibriersystem (400), umfassend:
- mindestens ein erstes optisches Detektierungsmittel (410), das an der Gantry (300) montiert ist,
- mindestens ein zweites optisches Detektierungsmittel (420), das in einem Umgebungsbereich der Liege (200) und/oder der Gantry (300) fixiert ist,
- einen Satz von ersten Passermarkern (412), die selektiv an definierten Positionen an der Liege (200) befestigbar sind und so ausgestaltet sind, dass sie durch das erste optische Detektierungsmittel (410) detektierbar sind, und
- ein Phantom (430), das selektiv an einer definierten Position an der Liege (200) befestigbar ist, wobei das Phantom (430) einen Satz von zweiten Passermarkern (421), die so ausgestaltet sind, dass sie durch das zweite optische Detektierungsmittel (420) detektierbar sind, und einen Satz von dritten Passermarkern (331, 341) umfasst, die so ausgestaltet sind, dass sie durch die Strahlungsbildgebungsvorrichtung (330, 340) detektierbar sind, und
- ein Steuermittel (500), das ausgestaltet ist, um die Strahlungsquelle (320) selektiv zu aktivieren und die Gantry (300) zu rotieren, und um für eine oder mehrere Rotationspositionen der Gantry (300) einen Schnittpunkt einer Strahlachse der Strahlungsquelle (320) und der Gantry-Achse (310) zu bestimmen, indem Detektierungsdaten von mindestens dem ersten optischen Detektierungsmittel (410), dem zweiten optischen Detektierungsmittel (420) und/oder der Strahlungsbildgebungsvorrichtung (330, 340) verknüpft werden.

2. Strahlentherapiesystem (100) nach Anspruch 1, wobei das Steuermittel (500) des Weiteren ausgestaltet ist, um eine Position und/oder Orientierung der Liege (200) zu steuern, um sie mit dem bestimmten Schnittpunkt auszurichten.

3. Strahlentherapiesystem (100) nach Anspruch 1 oder 2, wobei das Steuermittel (500) des Weiteren ausgestaltet ist, um basierend auf einem Bild des Phantoms (430), das durch die mindestens eine Strahlungsbildgebungsvorrichtung (330, 340) erfasst wurde, einen Strahlformer der Strahlungsquelle (320) an jeder Rotationsposition der Gantry (300) zu kalibrieren.

4. Strahlentherapiesystem (100) nach einem der vorhergehenden Ansprüche, wobei das Steuermittel (500) des Weiteren ausgestaltet ist, um basierend auf einem Bild des Phantoms (430), das durch die mindestens eine Strahlungsbildgebungsvorrichtung (330, 340) erfasst wurde, die Strahlungsbildgebungsvorrichtung (330, 340) an jeder Rotationsposition der Gantry (300) zu kalibrieren.

5. Strahlentherapiesystem (100) nach einem der vorhergehenden Ansprüche, wobei das Steuermittel (500) des Weiteren ausgestaltet ist, um basierend auf einem Bild des Phantoms (430), das durch die mindestens eine Strahlungsbildgebungsvorrichtung (330, 340) erfasst wurde, einen Versatz der Gantry (300) an jeder Rotationsposition der Gantry (300) zu bestimmen.

6. Strahlentherapiesystem (100) nach einem der vorhergehenden Ansprüche, wobei die dritten Passermarker (331, 341) in ein Material des Phantoms (430) eingebettet sind, um durch Verwendung der mindestens einen Strahlungsbildgebungsvorrichtung (330, 340) sichtbar zu sein.

7. Strahlentherapiesystem (100) nach einem der vorhergehenden Ansprüche, wobei das Phantom (430) mit dem mindestens einen ersten optischen Detektierungsmittel (410) verknüpft ist.

8. Strahlentherapiesystem (100) nach einem der vorhergehenden Ansprüche, wobei das Phantom (430) über das zweite optische Detektierungsmittel (420) mit mindestens einer absoluten Position in einem freien dreidimensionalen Raum verknüpft ist.

9. Strahlentherapiesystem (100) nach einem der vorhergehenden Ansprüche, wobei das Phantom (430) des Weiteren einen weiteren Satz von ersten Passermarkern (412) umfasst, die ausgestaltet sind, um durch das mindestens eine erste optische Detektierungsmittel (410) detektiert zu werden.

10. Strahlentherapiesystem (100) nach einem der vorhergehenden Ansprüche, wobei ein weiteres erstes optisches Detektierungsmittel (410) an der mindestens einen Strahlungsbildgebung (330, 340) montiert ist, um gegenüber dem ersten optischen Detektierungsmittel (410) angeordnet zu werden, welches an der Gantry (300) montiert ist.

11. Strahlentherapiesystem (100) nach einem der vorhergehenden Ansprüche, wobei des Weiteren erste Passermarker (412) selektiv an der Unterseite der Liege (200) befestigbar sind.

12. Strahlentherapiesystem (100) nach einem der vorhergehenden Ansprüche, wobei erste Passermarker (412) durch mindestens eine Rahmenstruktur (411) getragen werden, die an der Oberseite der Liege (200) an unterschiedlichen Befestigungspositionen (201) montiert werden kann.

13. Strahlentherapiesystem (100) nach einem der vorhergehenden Ansprüche, wobei das erste optische Detektierungsmittel (410) eine IR-Kamera ist.

14. Strahlentherapiesystem (100) nach einem der vorhergehenden Ansprüche, wobei das zweite optische Detektierungsmittel (420) eine Laservorrichtung ist.

15. Strahlentherapiesystem (100) nach einem der vorhergehenden Ansprüche, wobei ein weiterer Satz der ersten Passermarker (412) mittels einer Trägerstruktur (413) bereitgestellt wird, die an einem Patienten befestigbar ist, und wobei diese ersten Passermarker (412) mit den ersten Passermarkern (412) verknüpft sind, die an der Liege (200) befestigt sind.

16. Strahlentherapiesystem (100) nach einem der vorhergehenden Ansprüche, wobei das Steuermittel (500) des Weiteren ausgestaltet ist, um für die eine oder mehreren Rotationspositionen der Gantry (300) eine aktuelle oder prognostizierte Liegenposition zu bestimmen und mittels des ersten optischen Detektierungsmittels (410) und des ersten Passermarkers (412) eine aktuelle oder prognostizierte Position der Gantry (300) relativ zu der bestimmten aktuellen oder prognostizierten Position der Liege (200) zu bestimmen.

17. Strahlentherapiesystem (100) nach einem der vorhergehenden Ansprüche, wobei das Steuermittel (500) des Weiteren ausgestaltet ist, um basierend auf dem Bestimmen eines mechanischen Zentrums der Gantry (300) mittels des ersten optischen Detektierungsmittels (410) eine aktuelle oder prognostizierte Winkelgeschwindigkeit und/oder Beschleunigung der Gantry (300) zu bestimmen.

18. Verfahren zum Betreiben eines Strahlentherapiesystems (100), umfassend die Schritte:
- Bereitstellen eines Satzes von ersten Passermarkern (412) an einer Liege (200) des Strahlentherapiesystems (100) an definierten Positionen,
- Bereitstellen eines Phantoms (430) an der Liege (200) an einer definierten Position, wobei das Phantom (430) einen Satz von zweiten Passermarkern (421) und einen Satz von dritten Passermarkern (331, 341) umfasst,
- Detektieren der ersten Passermarker (412) durch ein erstes optisches Detektierungsmittel (410), das an einer Gantry (300) montiert ist, die so ausgestaltet ist, dass sie um eine Gantry-Achse (310) rotierbar ist und eine Strahlungsquelle (320) aufweist,
- Detektieren der zweiten Passermarker (421) durch ein zweites optisches Detektierungsmittel (420), das in einem Umgebungsbereich der Liege (200) und/oder der Gantry (300) fixiert ist,
- Detektieren der dritten Passermarker (331, 431) durch eine Strahlungsbildgebungsvorrichtung (330, 340) des Strahlentherapiesystems (100),
- Steuern der Strahlungsquelle (320), so dass sie selektiv aktiviert wird, und Steuern der Gantry (320), so dass sie rotiert, und
- für eine oder mehrere Rotationspositionen der Gantry (300) Bestimmen eines Schnittpunkts einer Strahlachse der Strahlungsquelle (320) und der Gantry-Achse (310), indem Detektierungsdaten von mindestens dem ersten optischen Detektierungsmittel (410), dem zweiten optischen Detektierungsmittel (420) und/oder der Strahlungsbildgebungsvorrichtung (330, 340) verknüpft werden.

## Revendications

1. Système de radiothérapie (100), comprenant
- une table (200),
- un portique (300) qui est configuré pour être rotatif autour d'un axe de portique (310) et ayant une source de rayonnement (320),
- au moins un dispositif d'imagerie de rayonnement (330, 340),
- un système d'étalonnage (400) comprenant :
- au moins un premier moyen de détection optique (410) monté sur le portique (300),
- au moins un deuxième moyen de détection optique (420) fixé dans une zone environnante de la table (200) et/ou du portique (300),
- un ensemble de premières marques de repère (412) qui peuvent être fixées sélectivement sur la table (200) à des positions définies et configurés pour être détectables par les premiers moyens de détection optique (410), et
- un fantôme (430) qui peut être fixé de manière sélective sur la table (200) à une position définie, le fantôme (430) comprenant un ensemble de deuxièmes marques de repère (421) configurées pour être détectables par le deuxième moyen de détection optique (420) et un ensemble de troisièmes marques de repère (331, 341) configurées pour être détectables par le dispositif d'imagerie de rayonnement (330, 340), et
- un moyen de commande (500) qui est configuré pour activer sélectivement la source de rayonnement (320) et faire tourner le portique (300), et, pour une ou plusieurs positions de rotation du portique (300), pour déterminer un point d'intersection d'un axe de faisceau de la source de rayonnement (320) et de l'axe du portique (310) en reliant les données de détection d'au moins le premier moyen de détection optique (410), le deuxième moyen de détection optique (420) et/ou le dispositif d'imagerie de rayonnement (330, 340).

2. Système de radiothérapie (100) selon la revendication 1, le moyen de commande (500) étant en outre configuré pour commander une position et/ou une orientation de la table (200) pour l'aligner au niveau du point d'intersection déterminé.

3. Système de radiothérapie (100) selon la revendication 1 ou 2, le moyen de commande (500) étant en outre configuré pour, sur la base d'une image du fantôme (430) capturée par l'au moins un dispositif d'imagerie de rayonnement (330, 340), étalonner un conformateur de faisceau de la source de rayonnement (320) à chaque position de rotation du portique (300).

4. Système de radiothérapie (100) selon l'une quelconque des revendications précédentes, le moyen de commande (500) étant en outre configuré pour, sur la base d'une image du fantôme (430) capturée par l'au moins un dispositif d'imagerie de rayonnement (330, 340), étalonner le dispositif d'imagerie de rayonnement (330, 340) à chaque position de rotation du portique (300).

5. Système de radiothérapie (100) selon l'une quelconque des revendications précédentes, le moyen de commande (500) étant en outre configuré pour, sur la base d'une image du fantôme (430) capturée par l'au moins un dispositif d'imagerie de rayonnement (330, 340), déterminer un décalage du portique (300) à chaque position de rotation du portique (300).

6. Système de radiothérapie (100) selon l'une quelconque des revendications précédentes, les troisièmes marques de repère (331, 341) étant incorporées dans un matériau du fantôme (430) pour être visibles au moyen de l'utilisation de l'au moins un dispositif d'imagerie de rayonnement (330, 340).

7. Système de radiothérapie (100) selon l'une quelconque des revendications précédentes, le fantôme (430) étant relié à l'au moins un premier moyen de détection optique (410).

8. Système de radiothérapie (100) selon l'une quelconque des revendications précédentes, le fantôme (430), par l'intermédiaire du deuxième moyen de détection optique (420), étant relié à au moins une position absolue dans un espace tridimensionnel libre.

9. Système de radiothérapie (100) selon l'une quelconque des revendications précédentes, le fantôme (430) comprenant en outre un ensemble supplémentaire de premières marques de repère (412) configurées pour être détectés par l'au moins un premier moyen de détection optique (410).

10. Système de radiothérapie (100) selon l'une quelconque des revendications précédentes, un premier moyen supplémentaire de détection optique (410) étant monté au niveau de l'au moins un dispositif d'imagerie de rayonnement (330, 340) pour être agencé à l'opposé du premier moyen de détection optique (410) monté au niveau du portique (300).

11. Système de radiothérapie (100) selon l'une quelconque des revendications précédentes, des premières marques de repère supplémentaires (412) pouvant être fixées de manière sélective sur un côté inférieur de la table (200).

12. Système de radiothérapie (100) selon l'une quelconque des revendications précédentes, des premières marques de repère (412) étant portées par au moins une structure de cadre (411) qui peut être montée le long d'un côté supérieur de la table (200) à différentes positions de fixation (201).

13. Système de radiothérapie (100) selon l'une quelconque des revendications précédentes, le premier moyen de détection optique (410) étant une caméra IR.

14. Système de radiothérapie (100) selon l'une quelconque des revendications précédentes, le deuxième moyen de détection optique (420) étant un dispositif laser.

15. Système de radiothérapie (100) selon l'une quelconque des revendications précédentes, un ensemble supplémentaire de premières marques de repère (412) étant fournies par l'intermédiaire d'une structure de support (413) pouvant être fixée à un patient et ces premières marques de repère (412) étant liées aux premières marques de repère (412) fixées à la table (200) .

16. Système de radiothérapie (100) selon l'une quelconque des revendications précédentes, le moyen de commande (500) étant en outre configuré, pour la ou les positions de rotation du portique (300), pour déterminer une position actuelle ou prédite de la table et, par l'intermédiaire du premier moyen de détection optique (410) et des premières marques de repère (412), pour déterminer une position actuelle ou prédite du portique (300) par rapport à la position actuelle ou prédite déterminée de la table (200).

17. Système de radiothérapie (100) selon l'une quelconque des revendications précédentes, le moyen de commande (500) étant en outre configuré pour, sur la base de la détermination d'un centre mécanique du portique (300) par l'intermédiaire du premier moyen de détection optique (410), déterminer une vitesse angulaire et/ou une accélération actuelle ou prédite du portique (300).

18. Procédé de fonctionnement d'un système de radiothérapie (100), comprenant les étapes de :
- fourniture d'un ensemble de premières marques de repère (412) au niveau d'une table (200) du système de radiothérapie (100) à des positions définies,
- fourniture d'un fantôme (430) au niveau de la table (200) à une position définie, le fantôme (430) comprenant un ensemble de deuxièmes marques de repère (421) et un ensemble de troisièmes marques de repère (331, 341),
- détection des premières marques de repère (412) par un premier moyen de détection optique (410) monté sur un portique (300) qui est configuré pour être rotatif autour d'un axe de portique (310) et ayant une source de rayonnement (320),
- détection des deuxièmes marques de repère (421) par un deuxième moyen de détection optique (420) fixé dans une zone environnante de la table (200) et/ou du portique (300),
- détection des troisièmes marques de repère (331, 431) par un dispositif d'imagerie de rayonnement (330, 340) du système de radiothérapie (100),
- commande de l'activation sélective de la source de rayonnement (320) et commande de la rotation du portique (320), et
- pour une ou plusieurs positions de rotation du portique (300), détermination d'un point d'intersection d'un axe de faisceau de la source de rayonnement (320) et de l'axe du portique (310) en reliant les données de détection d'au moins le premier moyen de détection optique (410), le deuxième moyen de détection optique (420) et/ou le dispositif d'imagerie de rayonnement (330, 340).
